Europäisches Patentamt

European Patent Office

Office européen des brevets

(11) Publication number: **0 240 624**
**A1**

## (12) EUROPEAN PATENT APPLICATION

(21) Application number: **86302480.8**

(22) Date of filing: **03.04.86**

(51) Int. Cl.⁴: **C12M 1/00**

(43) Date of publication of application:
**14.10.87 Bulletin 87/42**

(84) Designated Contracting States:
**AT BE CH DE FR GB IT LI NL SE**

(71) Applicant: **OY A W ENBOM AB**
**P.O. Box 27**
**SF-00301 Helsinki 30(FI)**

(72) Inventor: **Vatunen, Markku Tapani**
**Hepokuja 7 B 62**
**SF-01200 Vantaa(FI)**

(74) Representative: **Williams, Trevor John et al**
**J.A. KEMP & CO. 14 South Square Gray's Inn**
**London WC1R 5EU(GB)**

(54) A device for dissolving organic waste by anaerobic fermentation.

㊼ The invention relates to a biogas reactor, which comprises a fermentation unit (1), which has several series connected sections (2, 3, 4, 5, 6) which are separated from each other with walls (16, 17, 18, 19) so that the walls do not have any direct liquid or gas conncection with each other; members (20) for the feeding of the sludge to be treated into the fermentation unit; members (10) for removing the formed biogas and residual sludge from each of the sections, as well as one or several heating ducts (12, 13) passing through the fermentation unit, in which the flue gases produced in the burning of biogas are advantageously used as heating medium.

Fig. 2

EP 0 240 624 A1

## A DEVICE FOR DISSOLVING ORGANIC WASTE BY ANAEROBIC FERMENTATION

This invention relates to a device for transforming organic waste by anaerobic fermentation, in particular cattle manure, into biogas and other useful products as for instance fertilizers.

The anaerobic dissolving of organic substances is a multiphase process. First, the hydrolytic bacteria dissolve the carbohydrates, fats and proteins into smaller organic compounds, as sugar, alcohol and organic acids. These hydrolytic products are further dissolved through the effect of the acid bacteria into acetic acid, carbon dioxide and hydrogen. The final dissolving is done by the methane bacteria. The formed gas, the so called biogas, mainly contains methane (c. 60 - 70%) and carbon dioxide as well as minor amounts of hydrogen, nitrogen and hydrogen sulphide.

One important way of using biogas is direct burning in a boiler, however it is also suitable as fuel in motors. Biogas is also particularly suitable as fuel in small local electricity generating power plants. The combustion gases produced in the burning of biogas are appropriate for use in greenhouse cultivation. The residual sludge formed in the production of biogas may be used directly as fertilizer or can be further refined into granular fertilizer for instance.

The main users of biogas reactors are among others major farms, industrial plants and municipal sewage disposal works. Among waste containing organic substances, which are dissoluble by anaerobic fermentation, may particularly be mentioned the manure sludges of hog farms, cow stables and poultry farms, as well as the sewage sludges of paper mills, sugar mills, potato plants etc.

The most common biogas reactors are horizontal or vertical cylindrical reactors, which usually are made of steel. Such reactors are generally manufactured in factories and subsequently transported to the site of operation. This naturally puts restrictions as to the size of the reactor. The service of prior known closed cylindrical reactors is difficult and the operation has to be totally interrupted during service, and when re-starting a new germ stock has to be inoculated in the reactor. The mixture of biogas and air is explosive, forming thus a moment of danger in the starting period, as the explosion of a big steel tank may have disastrous consequences.

Efforts have been made to improve the efficiency of biogas reactors by dividing the reactor into different sectors by means of partition walls. The sludge then flows from one reactor to the other underneath and above the partition wall and/or through an opening in the partition wall. Such a reactor is disclosed in the US patent specification 4 429 043. This reactor type also was affected by the above problems. Especially when treating pig manure in biogas reactors, foam and solid sludge particles rise to the surface of the sludge, which gradually dry as gas bubbles pass by, and form a hard surface layer, which encumbers the flow of gas and sludge in the reactor. In order to avoid the forming of such a hard surface layer, various floating substances have for instance been added to the surface of the sludge, the results have not however been satisfactory.

The methane fermentation rate depends on the temperature of the sludge. It is known that the preferred fermentation temperature range in a biogas reactor is 32-37°C or 55-70°C. In these temperatures, the methane bacteria are most effective. Acid bacteria do not however require so high temperatures. As stagnant sludge conducts heat poorly, the heating generally has to be enhanced by mixing, which nevertheless has a negative effect on the fermentation.

The purpose of the present invention is to eliminate the above disadvantages and to achieve a device, by which even great amounts of sludge may be efficiently treated, which is easily maintained, assembled on the spot in a simple way, and in which the sludge is ecoomically and efficiently heated. The invention also has the purpose of achieving a device, in which the forming of a solid layer on the surface of the sludge is prevented.

The main characteristics of the invention are presented in the enclosed claims.

The solution of the device according to the present invention is very simple and economical. For the heating of sludge, flue gases generated in the burning of biogas are advantageously used, which are conducted from one end to the other of the fermentation unit along one or several horizontal heating ducts. The flue gases are advantageously conducted counter-current with regard to the direction of flow of the sludge.

The fermentation unit consists of several, advantageously at least four successive fermentation sections. A fermentation section consists advantageously of a U-shaped concrete element, on top of which a separate hood-shaped cover part is provided, which is made of fiber glass, plastic or some other appropriate material. The fermentation unit is assembled on the spot from such fermentation sections, whereby a partition wall, preferably made of steel, is located between two adjacent sections. The size of a fermentation unit may easily be varied by choosing an appropriate number of fermentation sections. Owing to the detachable cover part, the service of the device is easy and in the event of a possible explosion, the cover part is blown into the air 'or breaks without any serious consequences. The fermentation sections are advantageously disposed in succession, forming a U-shaped system, whereby the first and the last section are located next to each other.

The fermentation unit advantageously has an integral sludge duct passing through the entire fermentation unit and opens up into each fermentation section. At the bottom of each section, possible with the exception of the first and the last section, a sludge inlet and outlet are provided, through which the sludge duct is connected with each section. The inlet level and outlet level of the sludge in each section is determined by means of tubes disposed around the said openings. The sludge duct has a barrier member at each section, between the sludge inlet opening and outlet opening. Usually the sludge duct is closed at the point of each section, whereby the sludge flow is forced to rise into the corresponding fermentation sections. Owing to this structure it is still possible to keep the sludge duct open at the point of one section, e.g. during service, whereby the sludge flow passes by this section.

The residual sludge is advantageously removed from the fermentation unit through the exhaust duct, which is connected with each section. According to one preferred embodiment, the residual sludge is conducted to a heat exchanger, where it preheats the sludge to be treated.

The biogas reactor according to the invention is advantageously placed under the ground, so that only the hood-shaped cover part is visible. This is particularly advantageous in winter conditions in order to avoid heat losses.

The invention is described more in detail below with reference to the enclosed drawing, in which

figure 1 represents a schematic view from above of a preferred embodiment of the invention,

figure 2 represents a section along the line A-A in figure 1 in a slightly greater scale, and

figure 3 represents a section along the line B-B in figure 1 in a slightly greater scale.

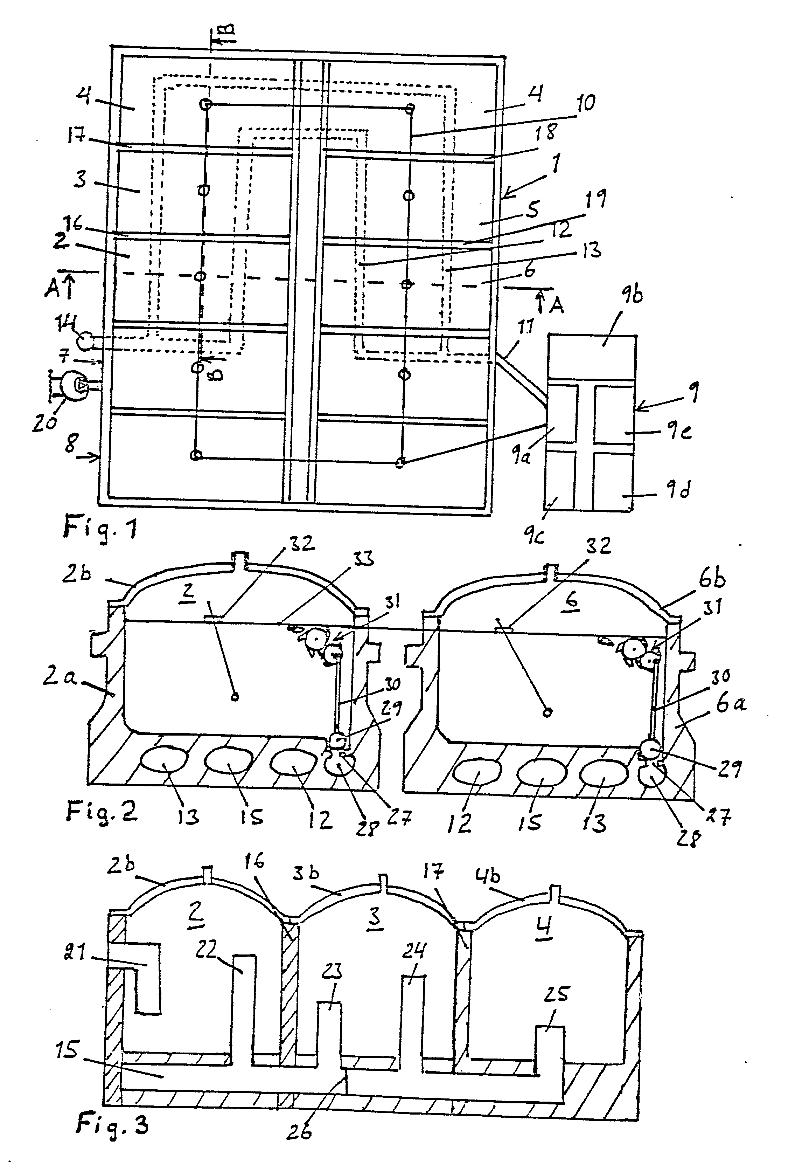

Figure 1 shows a device, which comprises the fermentation unit 1, the heat exchange unit 7 and the unit 8 for chemical treatment. The fermentation unit 1 consists of five series-connected fermentation sections 2, 3, 4, 5 and 6, which are so disposed, that the first 2 and fifth 6, and second 3 and fourth sections 5 are parallel. The fermentation sections are separated from each other with partition walls 16, 17, 18 and 19 so that they do not have any direct liquid nor gas connection with each other. The biogas formed in the methane fermentation process is conducted through the gas collecting pipage 10 to the electricity and heat generating unit 9, including a diesel motor 9a, a fuel reserve tank 9b, a generator 9c as well as the electricity and heat distribution centers 9d and 9e. The combustion gases from the diesel motor are conducted through the tube 11 into the two heating ducts 12 and 13, which pass through the entire fermentation unit 1 and further to the chimney 14.

Referring to figure 2 the fermentation units 2 and 6 as well as the other fermentation sections consist of U-shaped elements 2a and 6a, which advantageously are made of concrete. On top of these are attached e.g. by bolts the hoods 2b and 6b, which advantageously are made of glass fiber or plastic. The hoods are advantageously dark coloured, whereby the sun heats the sludge in the fermentation sections. This heating effect may be further enhanced by coating the inside of the hoods with aluminium foil. The water is condensed inside the hoods and the condensed water flows back to the sludge, whereby a relatively water-free biogas is obtained.

With reference to figures 1 and 3 the sludge to be treated is conducted through the feeding station 20 to the heat exchange unit 7 and further trough the pipe 21 to the first section 2 of the fermentation unit 1. When the level of the sludge in section 2 rises to the level of the upper end of the sludge discharge tube 22 it flows to the sludge duct 15 and from there to section 3 through the supply pipe 23. When the level of the sludge in sec tion 3 rises to the level of the upper end of the discharge pipe 24 it flows on through the sludge duct 15 and the supply pipe 25 to section 4. Correspondingly, the sludge flows from section 4 to section 5 and from there to section 6. In sludge duct 15, between the supply pipe and the discharge pipe a barrier member is provided, shown in figure 3 under the reference numeral 26. This barrier member forces the sludge flow into section 3, but e.g. in connection with the service this barrier member can be diverted so as to barr the supply pipe 23, whereby the sludge flows from section 2 directly to section 4.·

A quantity of residual sludge is continuosly discharged from the fermentation unit, which is equal to the quantity of fresh sludge fed into it. Referring to figure 2 each section has a residual sludge outlet 27, through which the residual sludge is aspirated by means of a pump into the residual sludge discharge duct 28, which passes through the entire fermentation unit. The outlet 27 is opened and closed by means of a rubber ball 29, which is moved upwards or downwards by means of the lever arm 30, which is connected with the catch 31. Figure 2 illustrates the rake 32, by which the forming of a solid layer on the surface of the sludge is prevented. The motor moves the rake back and forth through the intermediation of the wire rope 33. Each section has a rake, which is connected with the same wire rope. The rake cooperates with the above catch 31.

The residual sludge exhaust duct 28 is connected with the heat exchange unit 7, where it preheats the sludge to be treated. From the heat exchange unit the residual sludge is conducted to the unit 8 for chemical treatment. The final residual sludge is almost odourless and is well adapted to be used as a fertilizer.

As mentioned above, the flue gases are conducted to the heating ducts 12 and 13, whereby they heat the sludge in the fermentation sections. The flow rate of the flue gas and the retention time of the sludge in the fermentation unit as well as the effect of the heat exchanger are advantageously adjusted so that the temperature of the sludge in the last section 6 is c. 50-70°C and in the first section 2 c. 30-37°C. The temperature of the sludge to be fed is preferably maintained at c. 20°C, whereby the acid fermentation takes place already in the heat exchanger.

Only one advantageous embodiment of the invention has been describeda bove, however, it may of course be varied within the limits of the claims. Thus, the fermentation unit can for instance include the devices for circulating the germ stock.

## Claims

1. A device for transforming organic waste by anaerobic fermentation into biogas and other useful products, as fertilizers, which device comprises a fermentation unit (1), having several successive sections (2, 3, 4, 5, 6), members (20) for feeding the sludge to be treated into the fermentation unit, members (10, 28) for removing the formed biogas and residual sludge from the sections, **characterized** in that the sections (2, 3, 4, 5, 6) are separated with walls (16, 17, 18, 19) so that the sections do not have a direct liquid or gas conncection, in that the sections have members (15, 22, 23, 24, 25) for conducting the sludge from one section to another and in that through the fermentation section (1) passes one or several heating ducts (12, 13) in which as heating medium is advantageously used flue gases produced in the burning of biogas, and in that the device possibly comprises a heat exchange unit (7) for the preheating of the sludge to be fed into the fermentation unit.

2. A device according to claim 1, **characterized** in that the section consists of substantially U-shaped elements (2a, 6a) which are advantageously made of concrete, and of detachable hood-like covers (2b, 3b, 4b, 6b) which advantageously are made of fiber glass or plastic.

3. A device according to claim 1 or 2, **characterized** in that the fermentation unit (1) has a sludge duct (15) which leads to each of the sections.

4. A device according to claim 3, **characterized** in that the sections have sludge inlets and outlets, through the intermediation of which the sludge duct (15) is connected with the sections, and in that to the openings have been attached pipes (21, 22, 23, 24, 25) or corresponding members, by which is determined the level of the sludge supplied into the sections and the level of the sludge discharged from the sections, and in that the sludge duct (15) has a barr member (26) between the sludge inlet and the sludge outlet.

5. A device according to any of the preceding claims, **characterized** in that the fermentation unit (1) has a residual sludge exhaust duct (28) through the intermediation of which the outlet (27) in each section is connected with each section.

6. A device according to claim 5, **characterized** in that the residual sludge exhaust duct (28) is connected with a heat exchange unit (7) in which it gives heat to the sludge to be treated.

7. A device according to any one of the preceding claims, **characterized** in that each seciton is connected with a common gas collection pipage (10).

8. A device according to any one of the preceding claims, **characterized** in that in the sections are disposed rakes (32) or corresponding members moving back and forth, which prevent the forming of a solid layer on the surface of the sludge.

9. A device according to claim 8, **characterized** in that the rakes (32) or similar members cooperate with the barr member (29) of the resdiual sludge outlet.

10. A device according to any one of the preceding claims, **characterized** in that it additionally comprises a unit (8) for the chemical treatment of the residual sludge.

**Fig. 1**

**Fig. 2**

**Fig. 3**

**European Patent Office**

**EUROPEAN SEARCH REPORT**

Application number

EP 86 30 2480

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | EP-A-0 036 065 (E. GUNTER) <br><br> * Figure; Claims 1,2,5,7,9,14,17,18; page 12, lines 27-32; page 8, lines 20-34 * | 1,3-7, 10 | C 12 M 1/00 |
| | --- | | |
| A | WO-A-8 303 884 (P. SIEGEL) <br> * Figure 3; page 8, paragraph 2; page 9, line 10 * | 8,9 | |
| | --- | | |
| A | EP-A-0 102 279 (CHAMBRE DE L'AGRICULTURE DE L'AISNE) <br> * Figure; claims * | 1 | |
| | --- | | |
| A | US-A-2 429 589 (A. WILEY) <br> * Figure * | 1,7 | TECHNICAL FIELDS SEARCHED (Int. Cl.4) |
| | --- | | |
| A | BE-A- 494 781 (F. LAURENTY) <br> * Page 3, lines 30-34 * | 1 | C 12 M |
| | --- | | |
| A | FR-A-2 490 449 (R. MULLER) <br> * Figure 2 * | 2 | |
| | ----- | | |

The present search report has been drawn up for all claims

| Place of search | Date of completion of the search | Examiner |
|---|---|---|
| THE HAGUE | 23-01-1987 | COUCKE A.O.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82